# EUROPEAN PATENT SPECIFICATION

(11) **EP 3 085 383 B1**
(45) Date of publication and mention of the grant of the patent: **28.11.2018**
(21) Application number: 16166502.1
(22) Date of filing: 21.04.2016
(51) Int. Cl.: A61K 38/18, A61K 31/546, A61P 25/16, A61P 25/28

(54) **A PHARMACEUTICAL COMPOSITION COMPRISING ERYTHROPOIETIN AND CEFTRIAXONE AND A USE THEREOF IN THE MANUFACTURE OF A MEDICAMENT FOR THE TREATMENT OF PARKINSON'S DISEASE DEMENTIA**
PHARMAZEUTISCHE ZUSAMMENSETZUNG MIT ERYTHROPOIETIN UND CEFTRIAXON UND VERWENDUNG DAVON BEI DER HERSTELLUNG EINES MEDIKAMENTS ZUR BEHANDLUNG VON DEMENZ IM ZUSAMMENHANG MIT DER PARKINSON-KRANKHEIT
COMPOSITION PHARMACEUTIQUE COMPRENANT DE L'ÉRYTHROPOÏÉTINE ET DE LA CEFTRIAXONE ET SON UTILISATION DANS LA FABRICATION D'UN MÉDICAMENT POUR LE TRAITEMENT DE LA DÉMENCE LIÉE À LA MALADIE DE PARKINSON

(30) Priority: 22.04.2015 TW 104112897
(43) Date of publication of application: 26.10.2016
(73) Proprietor: Chung Shan Medical University, Taichung City (TW); Raygene Biotech International Inc., Tainan City (TW)
(72) Inventor: Ho, Ying-Jui, Taichung City (TW); Chen, Jian-Horng, Taichung City (TW)
(74) Representative: Lang, Christian

(56) References cited:
- US-A1- 2013 296 294
- HO SHIH-CHUN ET AL: "Effects of ceftriaxone on the behavioral and neuronal changes in an MPTP-induced Parkinson's disease rat model", BEHAVIOURAL BRAIN RESEARCH, vol. 268, 19 April 2015 (2015-04-19), pages 177-184, XP028651937, ISSN: 0166-4328, DOI: 10.1016/J.BBR.2014.04.022
- SERMIN GENC ET AL: "Erythropoietin restores glutathione peroxidase activity in 1-methyl-4-phenyl-1,2,5,6-tetrahydropyridi ne-induced neurotoxicity in C57BL mice and stimulates murine astroglial glutathione peroxidase production in vitro", NEUROSCIENCE LETTERS, vol. 321, no. 1-2, 1 March 2002 (2002-03-01), pages 73-76, XP055296499, IE ISSN: 0304-3940, DOI: 10.1016/S0304-3940(02)00041-1
- A. DHANUSHKODI ET AL: "A single intramuscular injection of rAAV-mediated mutant erythropoietin protects against MPTP-induced parkinsonism", GENES BRAIN AND BEHAVIOR, vol. 12, no. 2, 28 November 2012 (2012-11-28), pages 224-233, XP055296503, DK ISSN: 1601-1848, DOI: 10.1111/gbb.12001
- HUANG CHIU-KU ET AL: "Synergistic effects of ceftriaxone and erythropoietin on neuronal and behavioral deficits in an MPTP-induced animal model of Parkinson's disease deme", BEHAVIOURAL BRAIN RESEARCH, vol. 294, 18 August 2015 (2015-08-18), pages 198-207, XP029273298, ISSN: 0166-4328, DOI: 10.1016/J.BBR.2015.08.011

## Description

### BACKGROUND OF THE INVENTION

### 1. Field of the Invention

The present invention generally relates to a pharmaceutical composition comprising erythropoietin and ceftriaxone, and a use of the pharmaceutical composition, more particularly, to a use of the pharmaceutical composition in the manufacture of a medicament for the treatment of Parkinson's disease dementia.

### 2. Description of the Related Art

Parkinson's disease (PD) is a common neurodegenerative disease with motor symptoms such as resting tremor, rigidity, bradykinesia and postural instability. Moreover, about 25-50% of the PD patients may develop cognitive impairments on attention, working memory, short-term memory, executive function, recognition ability, constructional ability, visuospatial function and verbal fluency, which further leads to dementia (i.e. Parkinson's disease dementia, PDD). That is, PDD patients show syndromes including the aforesaid motor symptoms and cognitive impairments.

A conventional drug Levodopa (L-Dopa) commonly used clinically to ameliorate the motor symptoms of the PDD patients has a limited therapeutic effect on cognitive impairments. In addition, another conventional drug rivastigmine (brand name Exelon®) treating the cognitive impairments might cause deprivation of motor symptoms.

Genc et al. (Neurosci. Lett. 2002 Mar 15; 321(1-2): 73-6) discloses that in Parkinson's disease mice model erythropoietin increases the production and activity of glutathione peroxidase in astrocytes in the substantia nigra, thus increasing the antioxidative function of astrocytes. From Dhanushkodi et al. (Genes Brain Behav. 2013 Mar; 12(2): 224-33) it is further known that systemically administered rAAV-generated non-erythropoietic erythropoietin may protect against MPTP-induced parkinsonism by a combination of neuroprotection and enhanced axonal sprouting. Besides, ceftriaxone with a chemical structure shown in FIG. 1 is a drug for the treatment of PDD, which is disclosed by Taiwan Patent No. 201345529. Ceftriaxone is an antibiotic belonging to the third-generation cephalosporin and has broad-spectrum activity against Gram-positive bacteria and Gram-negative bacteria. Administration of ceftriaxone can treat cardinal motor symptoms as well as cognitive impairments.
For example, the US-application US 2013/0296294 A1 discloses the use of ceftriaxone at a daily dosage ranging from about 1.5 mg to about 35 mg per kilogram of a body weight of the human subject for treating Parkinson's disease dementia (PDD). Ho et al. (Behav. Brain Res. 2014 Jul 15; 268: 177-84) discloses that treatment with ceftriaxone at dosages of 100 and 200 mg/kg/day inhibits neurodegeneration in the hippocampus and nigrostriatal dopaminergic system and improves cognitive function in a MPTP-induced Parkinson's disease rat model. However, administration of high-dosage ceftriaxone may easily result in side effects.

In light of this, it is necessary to provide a new pharmaceutical composition which is very effective for the treatment of PDD in order to reduce side effects.

### SUMMARY OF THE INVENTION

It is therefore the objective of this invention to provide a pharmaceutical composition for the treatment of PDD.

One embodiment of the invention discloses a pharmaceutical composition for the treatment of PDD, including erythropoietin and ceftriaxone.

Another embodiment of the invention discloses a use of the pharmaceutical composition including erythropoietin and ceftriaxone in the manufacture of a medicament for the treatment of PDD. Erythropoietin and ceftriaxone are to be co-administrated to a subject in need thereof to reduce, alleviate, ameliorate, relieve or control the PDD syndromes.

In a preferred form shown, erythropoietin and ceftriaxone is to be concurrently administrated to the subject in need thereof, erythropoietin and ceftriaxone are to be sequentially administrated to the subject in need thereof, ceftriaxone and erythropoietin are to be sequentially administrated to the subject in need thereof, erythropoietin and ceftriaxone are to be separately administrated to the subject in need thereof, or ceftriaxone and erythropoietin are to be separately administrated to the subject in need thereof.

In a preferred form shown, erythropoietin is to be administrated to the subject in need thereof, followed by ceftriaxone being administrated to the subject in need thereof after 30 minutes.

In a preferred form shown, erythropoietin is to be administrated to the subject in need thereof with a dosage of 16.2-40.5 IU/kg/day, while ceftriaxone is to be administrated to the subject in need thereof with a dosage of 0.81-32.4 mg/kg/day.

In a preferred form shown, erythropoietin is to be administrated to the subject in need thereof by subcutaneous injection, intravenous injection, intramuscular injection, intraperitoneal injection, transdermal administration, sublingual administration or hebulization administration. Ceftriaxone is to be administrated to the subject in need thereof by intravenous injection, intramuscular injection, intraperitoneal injection, transdermal administration, sublingual administration or hebulization administration.

### BRIEF DESCRIPTION OF THE DRAWINGS

The present invention will become more fully understood from the detailed description given hereinafter and the accompanying drawings which are given by way of illustration only, and thus are not limitative of the present invention, and wherein:

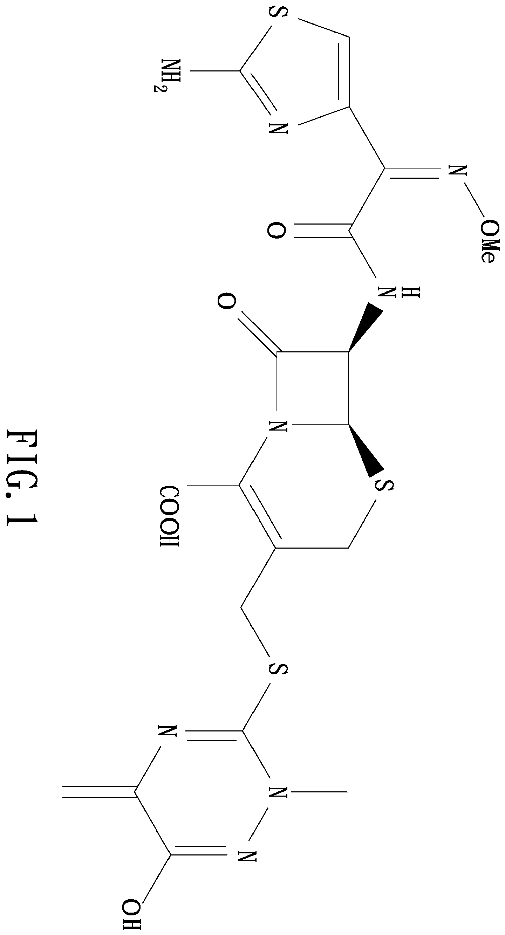

FIG. 1 depicts a diagram illustrating the chemical structure of ceftriaxone.
FIG. 2a depicts a schematic diagram illustrating the tendency detection run in trail (B).
FIG. 2b depicts a schematic diagram illustrating the forced run in trail (B).
FIG. 2c depicts a schematic diagram illustrating the choice run in trail (B).
FIG. 3 depicts a bar chart illustrating the percentage of correct response in trial (B).
FIG. 4a depicts a schematic diagram illustrating the open box used for the exposure session in trial (C).
FIG. 4b depicts a schematic diagram illustrating the open box used for the test session in trial (C).
FIG. 5 depicts a bar chart illustrating the percentage of time exploring object in trial (C).
FIG. 6 depicts a bar chart illustrating the density of dopaminergic (DAergic) neuron in trial (D).
FIG. 7 depicts a bar char illustrating the persentage of hippacampal CA1 area occupied by pyramidal neurons in trial (D).

In the various figures of the drawings, the same numerals designate the same or similar parts. Furthermore, when the term "first", "second", "third", "fourth", "inner", "outer", "top", "bottom" and similar terms are used hereinafter, it should be understood that these terms refer only to the structure shown in the drawings as it would appear to a person viewing the drawings, and are utilized only to facilitate describing the invention.

### DETAILED DESCRIPTION OF THE INVENTION

Erythropoietin (EPO) is a glycoprotein hormone that controls erythropoiesis. Erythropoietin is mainly produced by hepatic perisinusoidal cells in the fetal and perinatal period, and principally produced by renal interstitial fibroblasts during adulthood. In the present invention, administration of erythropoietin to the subject in need thereof includes, but not limited to, administration of a recombinant erythropoietin synthesized by a molecular biological technique to the subject in need thereof, as well as inducement of endogenous erythropoietin of the subject in need thereof *in vivo.* As an example, the subject in need thereof may stay in an oxygen-deficient environment, and the expression of endogenous erythropoietin increases due to the oxygen-deficient environment, which can be appreciated by a person having ordinary skill in the art.

In the present invention, erythropoietin and ceftriaxone can be co-administrated to the subject in need thereof, premitting the pharmacological effects of erythropoietin and ceftriaxone overlap each other, thereby reducing, alleviating, ameliorating, relieving or controlling the PDD syndromes of the subject in need thereof. Specifically, co-administration of erythropoietin and ceftriaxone includes the following ways. In the first way, erythropoietin and ceftriaxone can be concurrently administrated to the subject in need thereof, which means administration of erythropoietin and ceftriaxone to the subject in need thereof at the same time. In the second way, erythropoietin and ceftriaxone can be sequentially administrated to the subject in need thereof, which means after administering erythropoietin to the subject in need thereof, administering ceftriaxone to the subject in need thereof when the plasma drug concentration of erythropoietin remains a therapeutic drug concentration. For example, the time interval between administering erythropoietin and administering ceftriaxone is 10 minutes to 8 hours. In the third way, ceftriaxone and erythropoietin are sequentially administrated to the subject in need thereof, which means after administering ceftriaxone to the subject in need thereof, administering erythropoietin to the subject in need thereof when the plasma drug concentration of ceftriaxone remains a therapeutic drug concentration. For example, the time interval between administering ceftriaxone and administering erythropoietin is 10 minutes to 8 hours. In the forth way, erythropoietin and ceftriaxone can be separately administrated to the subject in need thereof, which means after administering erythropoietin to the subject in need thereof, administering ceftriaxone to the subject in need thereof when the plasma drug concentration of erythropoietin is below the therapeutic drug concentration. For example, the time interval between administering erythropoietin and administering ceftriaxone is 8-12 hours. In the fifth way, ceftriaxone and erythropoietin can be separately administrated to the subject in need thereof, which means after administering ceftriaxone to the subject in need thereof, administering erythropoietin to the subject in need thereof when the plasma drug concentration of ceftriaxone is below the therapeutic drug concentration. For example, the time interval between administering ceftriaxone and administering erythropoietin is 8-12 hours.

Moreover, erythropoietin and ceftriaxone can be administrated to the subject in need thereof via any suitable routes. For example, erythropoietin can be preferably administrated to the subject in need thereof by subcutaneous injection (SC injection), intravenous injection (IV injection), intraperitoneal injection (IP injection), intramuscular injection (IM injection), transdermal administration, sublingual administration or hebulization administration, while ceftriaxone can be administrated to the subject in need thereof by intravenous injection, intramuscular injection, intraperitoneal injection, transdermal administration, sublingual administration or hebulization administration.

In an embodiment, erythropoietin is administrated to the subject in need thereof in a dosage of 16.2-40.5 IU/kg/day, followed by administering ceftriaxone to the subject in need thereof in a dosage of 0.81-32.4 mg/kg/day after 30 minutes. However, the dosage of erythropoietin and/or ceftriaxone may vary according to the differences of the subject in need thereof, the sequence of administration and the routes of administration, which can be appreciated by a person having ordinary skill in the art.

According to the present invention, erythropoietin and ceftriaxone can be manufactured as a pharmaceutical composition. Moreover, erythropoietin and ceftriaxone can be concurrently, sequentially or separately administrated to the subject in need thereof by virtue of varying dosage form. In general, the pharmaceutical composition may include at least one pharmaceutical excipients. With such performance, the release of erythropoietin and/or ceftriaxone to the subject in need can be controlled. As an example, liposome can be used as the pharmaceutical excipient for coating one of the active substances (erythropoietin or ceftriaxone), assuring the extended releasing of the coated active substance, and therefore, the two active substances can be sequentially or separately administrated to the subject in need thereof.

In order to evaluate whether co-administration of erythropoietin and ceftriaxone can effectively treat the PDD syndromes of the subject in need thereof, the following trials are carried out.

### Trial (A). Induction of PDD Rats

Wistar male rats (12 week-old, weight ∼400 grams) purchased from the National Laboratory Animal Center (R.O.C.) are used. The rats are housed in an animal room with constant temperature of 24±1°C, where is kept on a 12-hours light and 12-hours dark cycle. The rats are housed and kept on free diet and water.

On the 1^{st} day, the rats are anesthetized, followed by bilaterally MPTP (1-methyl-4-phenyl-1,2,3,6-tetrahydropyridine hydrochloride)-infusion into the substantia nigra pars compacta (SNc). The rats with cognitive impairments on working memory and object recognition are used as the PDD rats (Ho et al., Behav Brain Res 268: 177-184, 2014).

Starting on the 1^{st} day, referring to TABLE 1, saline (group A1), erythropoietin (groups A2 & A3) and ceftriaxone (groups A4 & A5) are administrated to the PDD rats in the corresponding groups. Erythropoietin and ceftriaxone are sequentially administrated to the PDD rats of group A6 with a time interval of 30 minutes. Saline is also used to be administrated to the normal rats of group A0. The dosages of erythropoietin and/or ceftriaxone are shown in TABLE 1. Moreover, saline, erythropoietin and ceftriaxone are administrated for 14 days (from the 1^{st} day to the 14^{th} day).

**TABLE 1**

| Groups | MPTP induction | Erythropoietin (IU/kg/day) | Ceftriaxone (mg/kg/day) |
|---|---|---|---|
| A0 | - | 0 | 0 |
| A1 | + | 0 | 0 |
| A2 | + | 100 | 0 |
| A3 | + | 250 | 0 |
| A4 | + | 0 | 5 |
| A5 | + | 0 | 10 |
| A6 | + | 100 | 5 |

### Trial (B). T-Maze Test

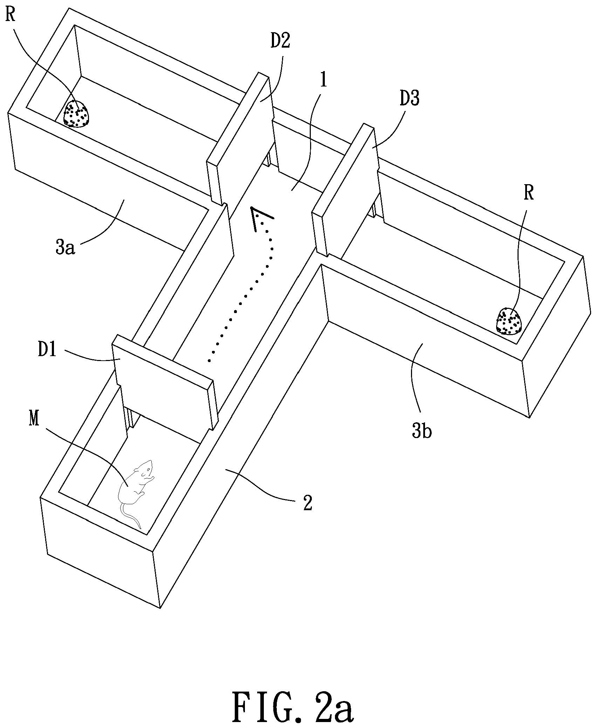

Working memory of the rats of groups A0-A6 is assessed using the T-maze test (Ho et al., Behav Brain Res 268: 177-184, 2014). Specifically, on the 8^{th} day, the T-maze shown in FIG. 2a is used for the tendency detection run. The T-maze includes a central square 1, a start arm 2 and two choice arms 3a & 3b opposite to each other. All of the start arm 2 and the two choice arms 3a & 3b connect the central square 1. The T-maze has openable doors D1, D2 & D3 in the start arm 2 and the two choice arms 3a & 3b, respectively. In the tendency detection run, the rats under test "M" are placed at the start arm 2, and the rewards "R" are placed at the terminal ends of the two choice arms 3a & 3b, respectively. After the doors D1, D2 & D3 respectively connects the start arm 2 and the two choice arms are open, the rat under test "M" will choose to enter the choice arm 3a or the choice arm 3b according to its turning tendency thereof. To make the following description clear, the rat under test "M" enters the choice arm 3a according to its turning tendency is used as an example.

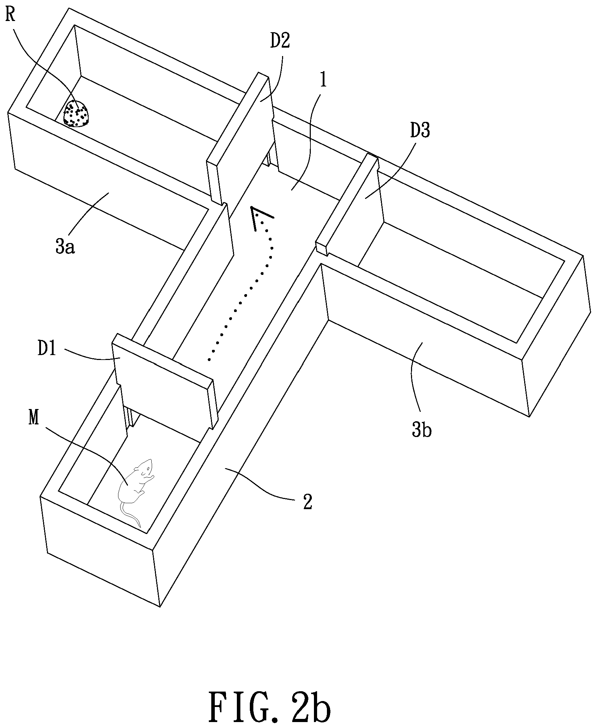

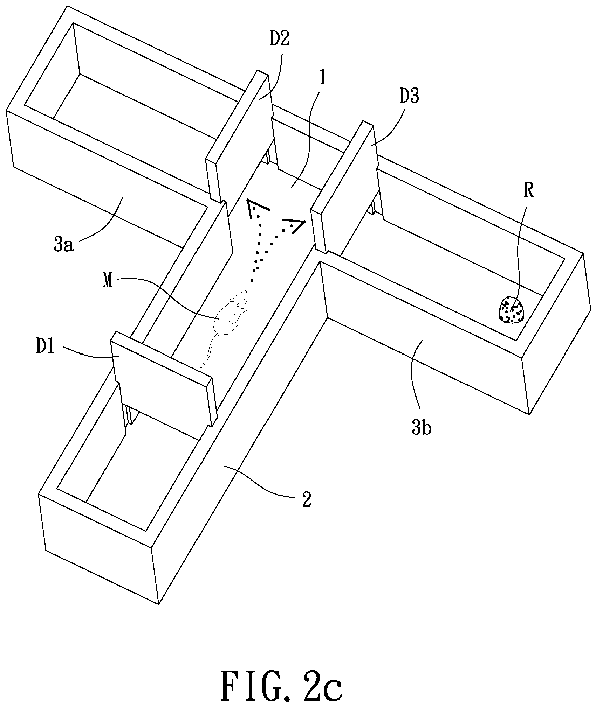

Then, a training session consisting 9 rounds, each of which is composed of a "forced run" and a "choice run", is carried out. Referring to FIG. 2b, in the forced run, the reward "R" is placed in the choice arm 3a, and the doors D1 and D2 are open (door D3 is closed), and therefore, the rat under test "M" can get the reward "R" when entering the choice arm 3a according to its turning tendency. Moreover, referring to FIG. 2c, in the choice run, the reward "R" is placed in the choice arm 3b, and the doors D1, D2 & D3 are open, and therefore, only in the case that the rat under test "M" can only get the reward "R" in the case of entering the choice arm 3b opposite to its turning tendency. On the 9^{th} day, the rat under test "M" undergoes the training session same as the training session on the 8^{th} day. By the training session carried out on the 8^{th} and 9^{th} days, the rat under test "M" will learn that in the case that both the doors D2 and D3 are open, the only way to get the reward "R" is to pass through the newly open door D3 and to enter the choice arm 3b opposite to its turning tendency.

On the 10^{th} day, a test session consisting 3 rounds, each of which is composed of a "forced run" and two "choice run", is carried out. The number of the rewards "R" that the rat under test "M" get (% of correct response) in the 6 choice runs is recorded.

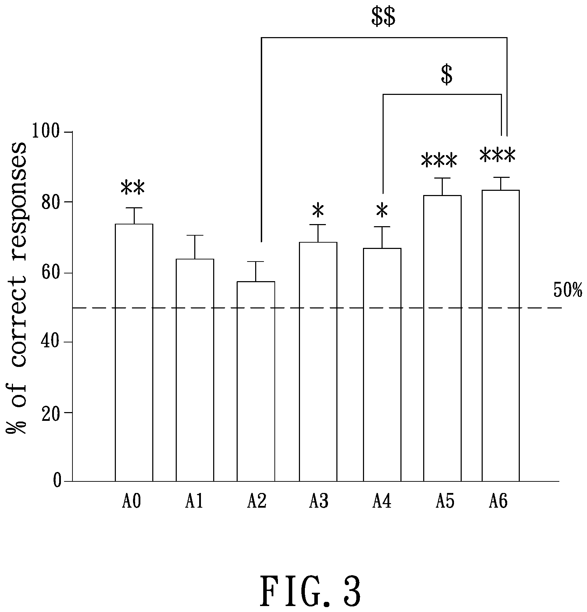

Referring to FIG. 3, the normal rat of group A0 shows a higher percentage of correct responses than chance (50%, meaning the rat under test "M" randomly enters the choice arm 3a or the choice arm 3b, *p*<0.01). The PDD rats of group A1 (saline) & A2 (erythropoietin, 100 IU/kg/day) have no significant difference between chance (50%), showing the aforesaid dosage is not sufficient to treat the cognitive impairment on working memory of the PDD rats. That is, the aforesaid dosage of erythropoietin (100 IU/kg/day) is an invalid dosage for treatment of the cognitive impairment on working memory.

Again, referring to FIG. 3, compared to chance (50%), the PDD rat of groups A3 (erythropoietin, 250 IU/kg/day) & A4 (ceftriaxone, 5 mg/kg/day) show a slightly treatment on the cognitive impairment on working memory (p<0.05), while the PDD rat of groups A5 (ceftriaxone, 10 mg/kg/day) & A6 (co-administration of 100 IU/kg/day of erythropoietin and 5 mg/kg/day of ceftriaxone) show a significant treatment on the cognitive impairment on working memory (p<0.001).

It is worthy to notice that compared to administration of high-dosage ceftriaxone (10 mg/kg/day), co-administration of low-dosage ceftriaxone (5 mg/kg/day) and invalid dosage erythropoietin (100 IU/kg/day) has a similar effect on the cognitive impairment on working memory, showing that co-administration with erythropoietin can effectively reduce the dosage of ceftriaxone needed. Moreover, compared to administration of low-dosage ceftriaxone (5 mg/kg/day), co-administration of invalid dosage erythropoietin (100 IU/kg/day) and low-dosage ceftriaxone (5 mg/kg/day) significantly treat the cognitive impairment on working memory of PDD rats (compared to group A2, *p*=0.002; compared to group A4: *p*=0.03), indicating that erythropoietin and ceftriaxone show synergistic effect on treating the cognitive impairment on working memory of PDD rats.

### Trial (C). Object Recognition Test

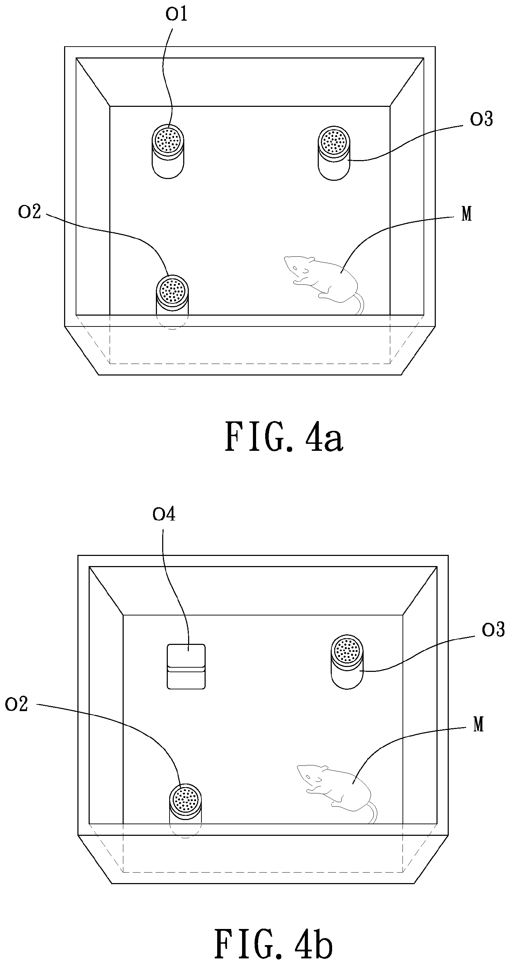

Recognition ability of the rats of groups A0-A6 is measured using the object recognition test (Ho et al., Behav Brain Res 268: 177-184, 2014). Specifically, on the 11^{th} & 12^{th} day, the rat under test "M" is placed in the open box, shown as FIG. 4a, for 5 minutes. Three flavorless objects (objects O1, O2 & O3) with the same size, color, shape and material are respectively fixed at three corners of the open box.

On the 13^{th} day, the rat under test "M" is first placed in the open box shown as FIG. 4a, and the time spent exploring the object O1 (T_{O1}) and the total time spent exploring the objects O1, O2 & O3 (T_{O1+O2+O3}) are recorded, respectively. The percentage of the exploration time spent on the object O1 is calculated as (T_{O1}/T_{O1+O2+O3})*100%. After 5 minutes, a novel object 04 with different size, color, shape and material is used to replace the object O1 (shown in FIG. 4b), and the rat under test "M" is placed in the open box. The time spent exploring the novel object 04 (T_{O4}) and the total time spent exploring the objects 02 & 03 and the novel object 04 (T_{O2+O3+O4}) are respectively recorded, and the percentage of the exploration time spent on the novel object 04 is calculated as (T_{O4}/T_{O2+O3+O4})*100%.

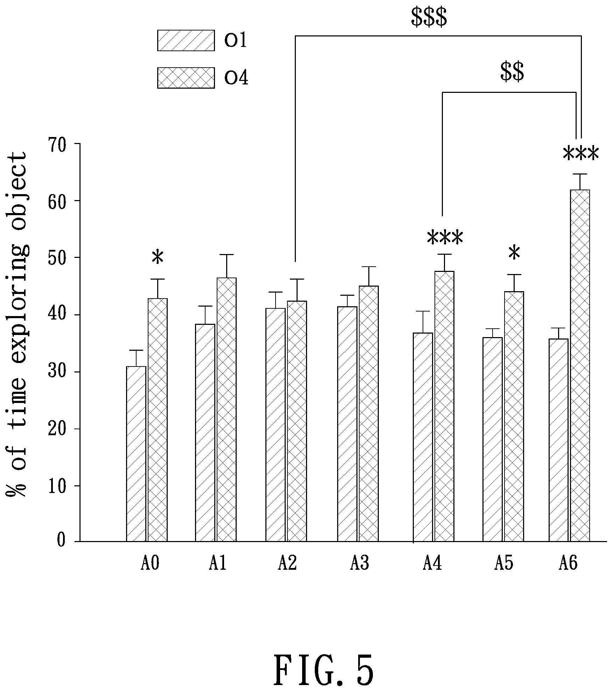

Referring to FIG. 5, the normal rat of group A0 significantly spends more time exploring the novel object 04 than exploring the object O1 (p<0.05), suggesting that the normal rat can recognize the novel object 04 in the environment. Moreover, according to the result of PDD rat of groups A1 (saline), the PDD rat has the cognitive impairment on recognition ability and cannot recognize the novel object 04. Moreover, administration of 100 IU/kg/day or 250 IU/kg/day of erythropoietin has no effect on treating the cognitive impairment on recognition ability of the PDD rat.

Again, referring to FIG 5, all of the PDD rats of groups A4 & A5 (ceftriaxone, 5 mg/kg/day or 10 mg/kg/day) and the PDD rat of group A6 (co-administration of 100 IU/kg/day of erythropoietin and 5 mg/kg/day of ceftriaxone) can recognize the novel object 04 in the environment (groups A4 & A6: p<0.001; group A5: p<0.05). Furthermore, compared to the PDD rat of group A4 (ceftriaxone, 5 mg/kg/day), the PDD rat of group A6 (co-administration of 100 IU/kg/day of erythropoietin and 5 mg/kg/day of ceftriaxone) spends more time exploring the novel object 04, demonstrating that the PDD rat of group A6 has a stronger recognition ability (p<0.01). In addition, compared to administration of low-dose ceftriaxone (5 mg/kg/day), co-administration of invalid dosage of erythropoietin (100 IU/kg/day) and low-dosage of ceftriaxone (5 mg/kg/day) can significantly treat the cognitive impairment on recognition ability of the PDD rat (compared to group A2: p=0.001; compared to group A4: p=0.004), also revealing that erythropoietin and ceftriaxone show synergistic effect on treating the cognitive impairment on recognition ability of PDD rat.

In conclusion, co-administration of erythropoietin and ceftriaxone shows synergistic effect on the cognitive impairment on working memory, as well as the cognitive impairment on recognition ability. Moreover, co-administration with erythropoietin can effectively decrease the dosage of ceftriaxone needed, not only effectively treating cognitive impairments of PDD patients, but also reducing side effects due to the high-dosage administration of ceftriaxone.

### Trial (D). Histopathological Analysis

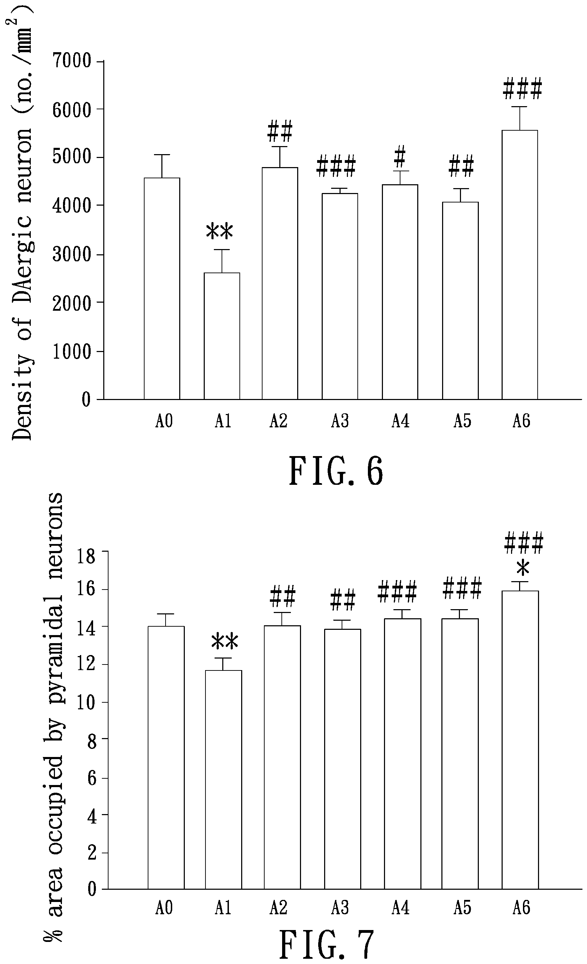

On the 14^{th} day, the rats of groups A0-A6 are sacrificed and the coronal sections are collected. The sections with SNc are used for tyrosine hydroxylase staining, while the sections with hippocampal CA1 area are used for Nissl staining. The density of DAergic neuron in the SNc and the percentage of the CA1 area occupied by the pyramidal neuron (Nissl-stained cells) are shown in FIGS. 6 & 7, respectively.

Referring to FIG. 6, compared to the normal rats of group A0, the PDD rats of group A1 has a decreased density of DAergic neuron in the SNc (p<0.01), indicating that the MPTP-induced PDD rat shows DAergic neurodegeneration. Compared to the PDD rat of group A1 (saline), the PDD rats of groups A2 & A3 (erythropoietin, 100 IU/kg/day or 250 IU/kg/day), the PDD rats of groups A4 & A5 (ceftriaxone, 5 mg/kg/day or 10 mg/kg/day) and the PDD rat of group A6 (co-administration of 100 IU/kg/day of erythropoietin and 5 mg/kg/day of ceftriaxone) show recovery of DAergic neuron density in the SNc (group A4: p<0.05; groups A2 & A5: p<0.01; groups A3 & A6: p<0.001).

Referring to FIG. 7, compared to the normal rat of group A0, the PDD rat of group A1 has a significantly decreased percentage of the CA1 area occupied by the pyramidal neuron (p<0.01), suggesting that the MPTP-induced PDD rats show pyramidal neurodegeneration. Moreover, compared to the PDD rat of group A1 (saline), the PDD rats of groups A2 & A3 (erythropoietin, 100 IU/kg/day or 250 IU/kg/day), the PDD rats of groups A4 & A5 (ceftriaxone, 5 mg/kg/day or 10 mg/kg/day) and the PDD rat of group A6 (co-administration of 100 IU/kg/day of erythropoietin and 5 mg/kg/day of ceftriaxone) show recovery of pyramidal neuron density in the CA1 area, recovering the percentage of the CA1 area occupied by the pyramidal neuron (groups A2 & A3: p<0.01; groups A4, A5 & A6: p<0.001). In addition, compared to the normal rat of group A0, the PDD rat of group A6 (co-administration of 100 IU/kg/day of erythropoietin and 5 mg/kg/day of ceftriaxone) shows a higher percentage of the CA1 area occupied by the pyramidal neuron (*p*<0.05), demonstrating the co-administration of erythropoietin and ceftriaxone has effect on recovery of pyramidal neuron density.

Besides, the aforesaid dosage can be converted into a dosage suitable for a human subject according to the dose translation formula based on body surface area (Shannon R.S. et al. (2007), FASEB J., 22: 659-661), suggesting that 16.2-40.5 IU/kg/day of erythropoietin and 0.81-32.4 mg/kg/day of ceftriaxone can cooperatively treat the PDD syndromes of the human subject thereof.

In conclusion, according to the cooperation of erythropoietin and ceftriaxone, the pharmaceutical composition for treating PDD according to the present invention can recover DAergic neuron density in the SNc and pyramidal neuron density in the CA1 area, treating the cardinal motor symptoms and cognitive impairments, such as the treatment on working memory or object recognition, in the PDD subject.

Moreover, the co-administration with erythropoietin can reduce the dosage of ceftriaxone needed, therefore can be rapidly and effectively metabolized by the metabolic organs such as liver and kidney, preventing ceftriaxone from accumulation in the organism. Furthermore, the reduced dosage of ceftriaxone can also decrease the burden to the metabolic organs such as liver and kidney, as well as diminish the risk of side effects.

## Claims

1. A pharmaceutical composition for use in the treatment of Parkinson's disease dementia (PDD) comprising erythropoietin and ceftriaxone.

2. The pharmaceutical composition for use in the treatment of PDD according to claim 1, **characterized in that** erythropoietin and ceftriaxone are to be co-administrated to a subject in need thereof to treat PDD syndromes of the subject in need thereof

3. The pharmaceutical composition for use in the treatment of PDD according to claim 2, wherein erythropoietin and ceftriaxone are concurrently to be administrated to the subject.

4. The pharmaceutical composition for use in the treatment of PDD according to claim 2, wherein erythropoietin and ceftriaxone are sequentially to be administrated to the subject.

5. The pharmaceutical composition for use in the treatment of PDD according to claim 4, wherein erythropoietin is to be administrated to the subject, followed by ceftriaxone being administered to the subject after 30 minutes.

6. The pharmaceutical composition for use in the treatment of PDD according to claim 5, wherein erythropoietin is to be administrated to the subject in a dosage of 16.2-40.5 IU/kg/day.

7. The pharmaceutical composition for use in the treatment of PDD according to claim 5, wherein ceftriaxone is to be administrated to the subject in a dosage of 0.81-32.4 mg/kg/day.

8. The pharmaceutical composition for use in the treatment of PDD according to claim 2, wherein ceftriaxone and erythropoietin are to be sequentially administrated to the subject.

9. The pharmaceutical composition for use in the treatment of PDD according to claim 2, wherein erythropoietin and ceftriaxone are to be separately administrated to the subject.

10. The pharmaceutical composition for use in the treatment of PDD according to claim 2, wherein ceftriaxone and erythropoietin are to be separately administrated to the subject.

11. The pharmaceutical composition for use in the treatment of PDD according to any of claims 2-10, wherein erythropoietin is to be administrated to the subject by subcutaneous injection, intravenous injection, intramuscular injection, intraperitoneal injection, transdermal administration, sublingual administration or hebulization administration.

12. The pharmaceutical composition for use in the treatment of PDD according to any of claims 2-10, wherein ceftriaxone is to be administrated to the subject by intravenous injection, intramuscular injection, intraperitoneal injection, transdermal administration, sublingual administration or hebulization administration.

## Patentansprüche

1. Pharmazeutische Zusammensetzung zur Verwendung bei der Behandlung einer durch die Parkinson-Krankheit bedingten Demenz (Parkinson's disease dementia, PDD), welche Erythropoietin und Ceftriaxon umfasst.

2. Pharmazeutische Zusammensetzung zur Verwendung bei der Behandlung von PDD nach Anspruch 1, welche **dadurch gekennzeichnet ist, dass** die gemeinsame Verabreichung von Erythropoietin und Ceftriaxon an eine diesbezüglich bedürftige Person vorgesehen ist, um PDD - Syndrome der diesbezüglich bedürftigen Person zu behandeln.

3. Pharmazeutische Zusammensetzung zur Verwendung bei der Behandlung von PDD nach Anspruch 2, bei welcher vorgesehen ist, dass der Person Erythropoietin und Ceftriaxon gleichzeitig verabreicht werden.

4. Pharmazeutische Zusammensetzung zur Verwendung bei der Behandlung von PDD nach Anspruch 2, bei welcher vorgesehen ist, dass der Person Erythropoietin und Ceftriaxon nacheinander verabreicht werden.

5. Pharmazeutische Zusammensetzung zur Verwendung bei der Behandlung von PDD nach Anspruch 4, mit welcher der Person zuerst Erythropoietin verabreicht wird und der Person darauffolgend nach 30 Minuten Ceftriaxon verabreicht wird.

6. Pharmazeutische Zusammensetzung zur Verwendung bei der Behandlung von PDD nach Anspruch 5, mit welcher der Person Erythropoietin in einer Dosis von 16,2-40,5 IU/kg/Tag verabreicht wird.

7. Pharmazeutische Zusammensetzung zur Verwendung bei der Behandlung von PDD nach Anspruch 5, mit welcher der Person Ceftriaxon in einer Dosis von 0,81-32,4 mg/kg/Tag verabreicht wird.

8. Pharmazeutische Zusammensetzung zur Verwendung bei der Behandlung von PDD nach Anspruch 2, bei welcher vorgesehen ist, dass der Person Ceftriaxon und Erythropoietin nacheinander verabreicht werden.

9. Pharmazeutische Zusammensetzung zur Verwendung bei der Behandlung von PDD nach Anspruch 2, bei welcher vorgesehen ist, dass der Person Erythropoietin und Ceftriaxon getrennt voneinander verabreicht werden.

10. Pharmazeutische Zusammensetzung zur Verwendung bei der Behandlung von PDD nach Anspruch 2, bei welcher vorgesehen ist, dass der Person Ceftriaxon und Erythropoietin getrennt voneinander verabreicht werden.

11. Pharmazeutische Zusammensetzung zur Verwendung bei der Behandlung von PDD nach einem der Ansprüche 2 bis 10, bei weicher die Verabreichung von Erythropoietin an die Person mittels subkutaner Injektion, intravenöser Injektion, intramuskulärer Injektion, intraperitonealer Injektion, transdermaler Verabreichung, sublingualer Verabreichung oder durch eine Verabreichung mittels Zerstäubung vorgesehen ist.

12. Pharmazeutische Zusammensetzung zur Verwendung bei der Behandlung von PDD nach einem der Ansprüche 2 bis 10, bei welcher die Verabreichung von Ceftriaxon an die Person mittels intravenöser Injektion, intramuskulärer Injektion, intraperitonealer Injektion, transdermaler Verabreichung, sublingualer Verabreichung oder durch eine Verabreichung mittels Zerstäubung vorgesehen ist.

## Revendications

1. Composition pharmaceutique à utiliser dans le traitement de la démence de la maladie de Parkinson (PDD) comprenant de l'érythropoïétine et de la ceftriaxone.

2. Composition pharmaceutique à utiliser dans le traitement de la PDD selon la revendication 1, **caractérisée en ce que** l'érythropoïétine et la ceftriaxone doivent être co-administrées à un sujet en ayant besoin pour traiter les syndromes de la PDD du sujet en ayant besoin.

3. Composition pharmaceutique à utiliser dans le traitement de la PDD selon la revendication 2, dans laquelle l'érythropoïétine et la ceftriaxone doivent être administrées concurremment au sujet.

4. Composition pharmaceutique à utiliser dans le traitement de la PDD selon la revendication 2, dans laquelle l'érythropoïétine et la ceftriaxone doivent être administrées séquentiellement au sujet.

5. Composition pharmaceutique à utiliser dans le traitement de la PDD selon la revendication 4, dans laquelle l'érythropoïétine doit être administrée au sujet, puis la ceftriaxone doit être administrée au sujet 30 minutes après.

6. Composition pharmaceutique à utiliser dans le traitement de la PDD selon la revendication 5, dans laquelle l'érythropoïétine doit être administrée au sujet dans un dosage de 16,2 à 40,5 UI/kg/jour.

7. Composition pharmaceutique à utiliser dans le traitement de la PDD selon la revendication 5, dans laquelle la ceftriaxone doit être administrée au sujet dans un dosage de 0,81 à 32,4 mg/kg/jour.

8. Composition pharmaceutique à utiliser dans le traitement de la PDD selon la revendication 2, dans laquelle la ceftriaxone et l'érythropoïétine doivent être administrées séquentiellement au sujet.

9. Composition pharmaceutique à utiliser dans le traitement de la PDD selon la revendication 2, dans laquelle l'érythropoïétine et la ceftriaxone doivent être administrées séparément au sujet.

10. Composition pharmaceutique à utiliser dans le traitement de la PDD selon la revendication 2, dans laquelle la ceftriaxone et l'érythropoïétine doivent être administrées séparément au sujet.

11. Composition pharmaceutique à utiliser dans le traitement de la PDD selon l'une quelconque des revendications 2 à 10, dans laquelle l'érythropoïétine doit être administrée au sujet par injection sous-cutanée, injection intraveineuse, injection intramusculaire, injection intrapéritonéale, administration transdermique, administration sublinguale ou administration par nébulisation.

12. Composition pharmaceutique à utiliser dans le traitement de la PDD selon l'une quelconque des revendications 2 à 10, dans laquelle la ceftriaxone doit être administrée au sujet par injection intraveineuse, injection intramusculaire, injection intrapéritonéale, administration transdermique, administration sublinguale ou administration par nébulisation.
